(19)

**Europäisches Patentamt**
**European Patent Office**
**Office européen des brevets**

(11) **EP 4 632 747 A1**

(12) **EUROPEAN PATENT APPLICATION**
published in accordance with Art. 153(4) EPC

(43) Date of publication:
**15.10.2025 Bulletin 2025/42**

(21) Application number: **23900825.3**

(22) Date of filing: **29.08.2023**

(51) International Patent Classification (IPC):
*G16B 40/20* (2019.01)　　*G16B 20/20* (2019.01)
*G16B 30/10* (2019.01)　　*G16B 45/00* (2019.01)
*C12Q 1/6886* (2018.01)　　*G16B 40/00* (2019.01)

(52) Cooperative Patent Classification (CPC):
**C12Q 1/6886; G16B 20/20; G16B 30/10;**
**G16B 40/00; G16B 40/20; G16B 45/00**

(86) International application number:
**PCT/KR2023/012761**

(87) International publication number:
**WO 2024/122804 (13.06.2024 Gazette 2024/24)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **05.12.2022　KR 20220168010**
　　　　　　　**21.02.2023　KR 20230022977**

(71) Applicant: **Theragen Bio Co., Ltd.**
**Gyeonggi-do 13488 (KR)**

(72) Inventors:
• **PAIK, Soon Myung**
**Seongnam-si, Gyeonggi-do 13488 (KR)**
• **HONG, Seong Eui**
**Seongnam-si, Gyeonggi-do 13488 (KR)**

• **HEO, Dong Hyuk**
**Seongnam-si, Gyeonggi-do 13488 (KR)**
• **KIM, In Young**
**Seongnam-si, Gyeonggi-do 13488 (KR)**
• **SEO, Hee Jae**
**Seongnam-si, Gyeonggi-do 13488 (KR)**
• **KIM, Seong Gwang**
**Seongnam-si, Gyeonggi-do 13488 (KR)**
• **KIM, Min Ji**
**Seongnam-si, Gyeonggi-do 13488 (KR)**
• **PARK, Ji In**
**Seongnam-si, Gyeonggi-do 13488 (KR)**
• **PARK, Hong Sil**
**Seongnam-si, Gyeonggi-do 13488 (KR)**

(74) Representative: **Müller Schupfner & Partner**
**Patent- und Rechtsanwaltspartnerschaft mbB**
**(Muc)**
**Bavariaring 11**
**80336 München (DE)**

(54) **METHOD AND DEVICE FOR DISTINGUISHING SOMATIC MUTATIONS AND ARTIFICIAL MUTATIONS IN DNA SEQUENCING DATA GENERATED FROM FORMALIN-FIXED PARAFFIN-EMBEDDED SAMPLES USING DEEP LEARNING**

(57)　　The present invention provides a method for providing information on the prediction of artificial or somatic mutations, and a device for providing information on the prediction of artificial or somatic mutations, wherein the method for providing information on the prediction of artificial or somatic mutations, which is implemented by a processor, comprises the steps of: receiving medical data or data obtained from biological samples derived from individuals; generating data necessary for distinguishing between artificial and somatic mutations on the basis of the received data; and predicting artificial or somatic mutations in the samples obtained from the individuals based on the generated data by using a neural network model configured to predict artificial or somatic mutations, with the necessary data for somatic mutation distinction inputted thereto.

EP 4 632 747 A1

Processed by Luminess, 75001 PARIS (FR)

[FIG. 1]

CORRESPONDING
TO SOMATIC
MUTATION

1000

300

200

100

DEVICE FOR
PROVIDING
INFORMATION ON
SOMATIC MUTATION

DEVICE FOR
PREDICTING
SOMATIC
MUTATION

SERVER FOR
PROVIDING DATA

## Description

### Technical Field

The present invention relates to a method and a device for distinguishing somatic mutation and artificial mutation in DNA sequencing data generated from formalin-fixed paraffin-embedded samples using deep learning.

### Background Art

Factors that affect the development of cancer include germline mutations, which include genetic tendency, and somatic mutation that occur in a somatic division process limited to specific organs or tissues.

The somatic mutation includes various types of mutations such as single nucleotide variation, structural variation, and aneuploidy. Specific cancer types are predicted by somatic single nucleotide mutations that occur when specific mutations and repair mechanisms fail due to exposure to chemicals, ultraviolet light, smoking, etc. A wide range of structural variation affects a normal gene function in cancer due to chromosomal deletion, insertion, inversion, tandem duplication, translocation, and complex rearrangement.

Major genes are involved in each cancer type, and if the variations of these genes are determined, the progression stage of cancer may be determined. In addition, even in cancer occurring in the same organ, since combinations of gene variations are different (heterogeneity), the prognosis, treatment, recurrence, and the like of cancer may be applied differently according to these other genetic differences.

Since the first analysis of the human genome in 2003, the development and application of genome sequencing technology have expanded in various ways. With the development of analytical technology capable of identifying many genetic mutations in a single test using next generation sequencing (NGS), cancer genome diagnosis is beginning to play an important role in effective cancer treatment. In conjunction with the success of developing targeted anticancer therapy for mutant proteins generated by somatic mutation in major genes such as EGFR and PIK3CA, the clinical demand for cancer genome diagnosis is increasing.

For genome analysis, ideally, DNA sequencing needs to be performed by extracting nucleic acids immediately from cancer tissue within 10 minutes of the disruption of vascular supply during surgery, or extracting nucleic acids if necessary while frozen in liquid nitrogen and stored at an extremely low temperature below -70°C. However, it is impossible to apply the method to surgical tissues of all patients who are not research targets in hospitals, and frozen tissues are not optimal samples as samples for histopathologic examination, and thus all human body-derived tissues are formalin-fixed and paraffin-embedded (FFPE), then microsectioned and subjected to histopathologic examination. FFPE blocks may be stored at room temperature for at least 10 years or permanently. The need for actual cancer genome analysis is determined after the results of pathologic examination using FFPE tissue, there is often no surplus tissue that may be cryopreserved after obtaining tissues required for diagnostic FFPE blocks due to the size of the cancer tissue that may be obtained from surgery or biopsy, and there are many hospitals without the infrastructure for cryopreservation, and thus most clinical genome analyses are conducted using FFPE samples.

The FFPE samples have the advantage of being convenient to store and optimal for histopathologic examination, but have the disadvantage for genomic analysis. That is, artificial mutation (artifacts) irrelevant to cancer are induced by chemical reactions occurring during a formalin fixing process, and during DNA sequencing using an NGS technique, up to 10-fold more mutations than original mutations are discovered. The presence or absence of gene mutations can be diagnosed with considerable accuracy during genome analysis by preparing a panel that targets only hot spot gene mutations of some important cancer genes. However, during analysis at an exome or genome level, it is difficult to distinguish between actual mutations and artificial mutation. The presence of artificial mutation causes inaccuracy in measuring Tumor Mutation Burden or discovering neoantigens, which becomes a major obstacle to realizing cutting-edge medicine, such as prediction of immunotherapy responses, development of neoantigen vaccines, and the like. So far, methods have been developed to remove artificial mutation from FFPE NGS DNA sequencing data, but clinical applicability is vulnerable by removing most actual mutations while removing artificial mutation, and thus the need for a technology of removing most artificial mutation while preserving actual mutations has been highlighted.

The background art of the invention has been prepared to further facilitate understanding of the present invention. It should not be understood that the matters described in the background art of the invention exist as prior arts.

### Disclosure

### Technical Problem

The present inventors recognized a problem that an absolute majority of general clinical samples are FFPE tissues, and NGS analysis is difficult due to the above-mentioned problem in the case of old FFPE tissues or samples mutated during a fixation process.

That is, the present inventors recognized that when distinguishing somatic mutation using existing FFPE samples, the accuracy was lowered due to artificial mutation caused by various factors, and thus a method capable of accurately predicting only somatic mutation was needed, and then it was noted that accuracy may be improved when using a deep-learning neural network model like the present invention.

Particularly, the present inventors analyzed paired NGS data generated from frozen tissue and FFPE tissue derived from the same tissue, defined mutations found only in the FFPE as artificial mutation, and performed deep learning with learning data including cosine similarity to recognize that it was possible to predict somatic mutation compared to artificial mutation with high accuracy.

As a result, the present inventors could confirm that it was possible to distinguish between artificial mutation and somatic mutation with high accuracy by applying a learned neural network model of the present invention.

As a result, the present inventors have developed an information provision system to distinguish between artificial mutation and somatic mutation generated by FFPE based on a deep-learning neural network model of the present invention.

Accordingly, the present inventors were able to recognize that the limitations of conventional diagnosis and information provision systems may be supplemented by introducing an information provision system that distinguishes between artificial mutation and somatic mutation generated by FFPE based on the deep-learning neural network model of the present invention, and thus, an efficient treatment method may be suggested.

Therefore, an object of the present invention is to provide a method for providing information on the prediction of somatic mutation through removal of artificial mutation and preservation of somatic mutation samples based on deep learning of the present invention, and a device using the same.

The objects of the present disclosure are not limited to the aforementioned objects, and other objects, which are not mentioned above, will be apparent to a person having ordinary skill in the art from the following description.

**Technical Solution**

One aspect of the present invention provides a method for providing information on the prediction of somatic mutation based on deep learning according to an embodiment of the present invention.

The method for providing information is implemented by a processor and includes receiving medical data and/or data on mutations obtained from biological samples derived from individuals; generating data necessary for distinguishing between artificial and somatic mutations based on the received data; and predicting artificial or somatic mutations in the samples obtained from the individuals, by using a neural network model configured to predict artificial or somatic mutations, with the necessary data for distinguishing the somatic mutation, using the calculated data as an input.

At this time, the medical data and/or the data obtained from the biological samples derived from the individuals may be data on mutations, specifically somatic mutation. The medical data may be next generation sequencing (NGS) data, and specifically, a whole genome sequence (WGS) sequenced for a whole genome, a whole exome sequencing (WES) file, a targeted sequencing file, and the like. The biological samples derived from the individuals may be data on mutations observed in the samples, specifically somatic mutation observed in the samples, but are not limited thereto.

According to the feature of the present invention, the calculating of the data necessary for distinguishing between the artificial and somatic mutations based on the received data may further include vectorizing the received data and calculating a similarity based on the vectorized data. At this time, in the calculating of the similarity based on the vectorized data, the similarity may be a cosine similarity, but is not limited thereto.

In the predicting of the artificial or somatic mutations in the samples obtained from the individuals, by using a neural network model configured to predict artificial or somatic mutations, with the necessary data for distinguishing the somatic mutation, using the calculated data as an input, the neural network model may be a deep neural network (DNN), but is not limited thereto.

According to the feature of the present invention, in the calculating of the data necessary for distinguishing between the artificial and somatic mutations based on the received data, the calculated data may include at least any one selected from the group consisting of the number of reads in which a reference sequence is observed/sequence depth at the corresponding position, the number of reads in which an alternate sequence is observed/sequence depth at the corresponding position, a ratio of a difference between the number of sequences in which read1 is mapped to a forward strand among the reads in which the reference sequence is observed and the number of sequences in which read2 is mapped to a reverse strand in the reads in which the reference sequence is observed, a ratio of a difference between the number of sequences in which read1 is mapped to a forward strand among the reads in which the alternate sequence is observed and the number of sequences in which read2 is mapped to a reverse strand in the reads in which the alternate sequence is observed, a value obtained by dividing a median of the inserted length in the read in which the alternate sequence is observed by a median of the inserted length in the read in which the reference sequence is observed, a length of double-sequenced bases by read1 and read2 in a reference fragment, the number of double-sequenced bases by read1 and read2 in the reference fragment, a length of double-sequenced bases by read1 and read2 in an alternate fragment, the number of double-sequenced bases by read1 and read2 in the alternate fragment, a reference sequence (REF_3_BASES) including one base before and after a mutation position, an alternate sequence (ALT_3_BASES) including one base before and after a mutation position, a ratio of a difference between the number of sequences to be mapped to the forward strand among the reads in which the reference sequence is observed and the number of sequences to be mapped to the reverse strand in the reads in which the reference sequence is observed, and a ratio of a difference between the number of sequences to be mapped to the forward strand among the reads in which the alternate sequence is observed and the number of sequences to be mapped to the reverse strand in the reads in which the alternate sequence is observed. **In** addition, the calculated data may further include a cosine similarity between vectors defined by (the number of sequences in which read1 is mapped to a forward strand among reads in which the reference sequence is observed and the number of sequences in which read2 is mapped to a reverse strand), or a cosine similarity between a vector defined by (the number of sequences to be mapped to the forward strand among reads in which the reference sequence is observed and the number of sequences to be mapped to the reverse strand) and a vector defined by (the number of sequences to be mapped to the forward strand among reads in which the alternate sequence is observed and the number of sequences to be mapped to the reverse strand). However, the present invention is not limited thereto.

In an embodiment of the present invention, the cosine similarity may be represented by the following Equation 1.

[Equation 1]

$$\text{similarity} = \cos(\theta) = \frac{\mathbf{A} \cdot \mathbf{B}}{\|\mathbf{A}\|\|\mathbf{B}\|} = \frac{\sum_{i=1}^{n} A_i B_i}{\sqrt{\sum_{i=1}^{n} A_i^2}\sqrt{\sum_{i=1}^{n} B_i^2}}$$

According to the feature of the present invention, in the predicting of the artificial or somatic mutations in the samples obtained from the individuals by using a neural network model configured to predict artificial or somatic mutations, with the necessary data for distinguishing the somatic mutation, inputted there using the calculated data as an input, the sample obtained from the individual may be an FFPE sample, the individual may be an individual developed with cancer, and the cancer may be any one of liver cancer, colon cancer, breast cancer, lung cancer, and fibrosarcoma, but is not limited thereto.

Another aspect of the present invention provides a system for providing information including a device for providing information on the prediction of somatic mutation according to the embodiment of the present invention, a device for predicting mutations, and a server for providing medical data.

Details of other embodiments will be included in the detailed description of the invention and the accompanying drawings.

**Advantageous Effects**

According to the present invention, it is possible to provide information on the prediction of somatic mutation to enable tailored personal medicine by providing information on the prediction of artificial or somatic mutations using a deep-learning neural network model.

Particularly, it is possible to more accurately predict artificial or somatic mutations by calculating data associated with somatic mutation and determining prediction results for artificial or somatic mutations based on the data.

That is, it is possible to more accurately perform setting of treatment directions for individuals and contribute to selection of tailored treatment methods by using information on the prediction of artificial or somatic mutations based on a deep-learning neural network model.

According to the present invention, it is possible to complement the limitations of using conventional FFPE samples by applying a deep-learning neural network model trained with learning data including cosine similarity for prediction of artificial or somatic mutations to predict artificial mutation and somatic mutation with high accuracy.

The effects of the present invention are not limited by the foregoing, and other various effects are anticipated herein.

**Description of Drawings**

FIG. 1 illustrates a system for providing information on the prediction of artificial or somatic mutations using a device for predicting artificial or somatic mutations according to an embodiment of the present invention.

FIG. 2 illustrates an example of a configuration of a device for predicting artificial or somatic mutations according to an embodiment of the present invention.

FIG. 3 is a block diagram of a process of a method for providing information on the prediction of artificial or somatic mutations according to an embodiment of the present invention.

FIG. 4 exemplarily illustrates a process of a method for providing information on artificial or somatic mutations according to various embodiments of the present invention.

FIG. 5a is a diagram illustrating results of evaluating the prediction performance of a deep-learning neural network model of the present invention.

FIG. 5b is a diagram illustrating results of evaluating the prediction performance of Comparative Example 1 (FFpolish pre-trained).

FIG. 5c is a diagram illustrating results of evaluating the prediction performance of Comparative Example 2 (FFpolish re-trained).

FIG. 5d is a diagram illustrating results of evaluating the prediction performance of Comparative Example 3 (SOBDetector).

FIG. 6a illustrates a comparison of the accuracy of predicting artificial or somatic mutations for liver cancer samples using a deep-learning neural network model of the present invention.

FIG. 6b illustrates a comparison of the accuracy of predicting artificial or somatic mutations for colon cancer samples using a deep-learning neural network model of the present invention.

FIG. 6c illustrates a comparison of the accuracy of predicting artificial or somatic mutations for breast cancer samples using a deep-learning neural network model of the present invention.

FIG. 6d illustrates a comparison of the accuracy of predicting artificial or somatic mutations for lung cancer samples using a deep-learning neural network model of the present invention.

FIG. 6e illustrates a comparison of the accuracy of predicting artificial or somatic mutations for fibrosarcoma samples using a deep-learning neural network model of the present invention.

FIG. 7a illustrates a comparison of the accuracy of predicting artificial or somatic mutations for liver cancer samples using Comparative Example (FFpolish pre-trained).

FIG. 7b illustrates a comparison of the accuracy of predicting artificial or somatic mutations for colon cancer samples using Comparative Example (FFpolish pre-trained).

FIG. 7c illustrates a comparison of the accuracy of predicting artificial or somatic mutations for breast cancer samples using Comparative Example (FFpolish pre-trained).

FIG. 7d illustrates a comparison of the accuracy of predicting artificial or somatic mutations for lung cancer samples using Comparative Example (FFpolish pre-trained).

FIG. 7e illustrates a comparison of the accuracy of predicting artificial or somatic mutations for fibrosarcoma samples using Comparative Example (FFpolish pre-trained).

**Modes of the Invention**

Advantages and features of the present invention, and methods for accomplishing the same will be more clearly understood from embodiments described in detail below with reference to the accompanying drawings.

However, the present invention is not limited to the embodiments set forth below, and will be embodied in various different forms. The embodiments are just for rendering the disclosure of the present invention complete and are set forth to provide a complete understanding of the scope of the invention to a person with ordinary skill in the technical field to which the present invention pertains, and the present invention will only be defined by the scope of the claims. In connection with the description of drawings, like reference numerals may be used for like components.

In this specification, expressions such as "have," "may have," "include," or "may include" refer to the presence of the corresponding feature (e.g., numerical value, function, operation, or component such as part), and does not exclude the presence of additional features.

In the present specification, the expression such as "A or B", "at least one of A and/or B", or "one or more of A and/or B" may include all possible combinations of items listed together. For example, "A or B", "at least one of A and B", or "at least one of A or B" may refer to all cases of (1) including at least one A, (2) including at least one B, or (3) including both at least one A and at least one B.

Expressions such as "first," and "second," used herein may describe various components regardless of the order and/or importance, and will be used only to distinguish one component from the other component, but are not limit the corresponding components. For example, a first user device and a second user device may represent different user devices, regardless of the order or importance. For example, a first component may be referred to as a second component, and similarly, the second component may also be referred to as the first component without departing from the scope of the present specification.

When a certain component (e.g., a first component) is referred to as being "(operatively or communicatively) coupled with/to" or "connected to" the other component (e.g., a second component), it will be understood that the component may be directly connected to the other component, or may be connected to the other component through another component (e.g., a third component). When a certain component (e.g., a first component) is referred to as being "directly coupled with/to" or "directly connected to" the other component (e.g., a second component), it can be understood that another component (e.g., a third component) does not exist between the certain component and the other component.

The expression of "configured to" used herein may be changed and used to, for example, "suitable for", "having the capacity to", "designed to", "adapted to", "made to" or "capable of", depending on the situation. The term of "configured to" may not necessarily mean "specially designed to" in hardware. In some situations, the expression "a device configured to" may mean that the device "capable of" together with other devices or parts. For example, the phrase "a processor configured to perform A, B, and C" may mean a dedicated processor (e.g., embedded processor) for performing the corresponding operation, or a generic-purpose processor (e.g., CPU, GPU or application processor) capable of performing the corresponding operations by executing one or more software programs stored in a memory device.

The terms used herein are used to describe only specific embodiments, and may not be intended to limit the scope of other embodiments. A singular expression may include a plural expression unless the context clearly dictates otherwise. The terms used herein, including technical or scientific terms, may have the same meaning as generally understood by those of ordinary skill in the art described in the present specification. The terms defined in a general dictionary among the terms used herein may be interpreted in the same or similar meaning as or to the meaning on the context of the related art, and will not be interpreted as an ideal or excessively formal meaning unless otherwise defined in the present specification. In some cases, even the terms defined in the present specification can not be interpreted to exclude the embodiments of the present specification.

The features of various embodiments of the present invention may be partially or entirely coupled or combined with each other and may be interlocked and operated in technically various ways to be sufficiently appreciated by those skilled in the art, and the embodiments may be implemented independently of or in association with each other.

For the clarity of the interpretation of the present specification, hereinafter, the terms used in the present specification will be defined.

As used herein, the term "individual" may mean any object for which mutation, specifically somatic mutation, is to be predicted. For example, the individual may be an individual in which somatic mutation has occurred. At this time, the individual disclosed in the present specification may be all mammals except for humans, but is not limited thereto.

As used herein, the term "biological sample" refers to all biological samples that are derived from individuals and can be stored in an FFPE format, and may include urine, tissue, cell lysate, whole blood, plasma, serum, saliva, ocular fluid, cerebrospinal fluid, sweat, milk, ascites fluid, synovial fluid, peritoneal fluid, etc., but is not limited thereto. Preferably, the biological sample may be a tissue sample derived from the individuals to determine whether there are somatic mutation, but is not limited thereto. Specifically, the biological sample of the present invention may be a formalin-fixed paraffin-embedded (FFPE) sample, and the FFPE sample may mean a sample capable of being stored at room temperature by fixing a tissue in formalin by a standard method used when collecting and storing cancer tissue from a patient, hardening the tissue with paraffin to prepare a paraffin block, thereby enabling storage at room temperature, and used for various histological examinations by slicing the paraffin block thinly multiple times.

As used herein, the term "somatic mutation (somatic variant)" means that one of the characteristics of cancer identified in cells accompanies genetic abnormalities, and thus, it is possible to distinguish the occurrence and type of cancer by identifying somatic variants. Accordingly, in the present invention, medical data and/or data obtained from biological samples derived from individuals may be data on somatic mutation, and the medical data may be next generation sequencing (NGS) data, but is not limited thereto, and may mean observing somatic mutation in biological samples derived from individuals, but is not limited thereto.

As used herein, the term "artificial mutation (artifact)" may mean an artificial defect, etc. that occurs due to physical or chemical alteration to cause non-cancer alteration, but is not limited thereto.

As used herein, the term "Next Generation Sequencing (NGS)" refers to next-generation sequencing, which is one of high-speed sequencing methods for the base sequence of a genome.

At this time, the NGS may be applied to genome and whole-genome analysis to achieve various purposes including clinical research. Meanwhile, most NGS analyses may be performed simultaneously on a plurality of target samples.

As used herein, the term "NGS data" may mean a file containing sequencing data analyzed for the target samples.

At this time, the NGS data may include a whole genome sequencing (WGS) file sequenced for a whole genome, a whole exome sequencing (WES) file, an RNA sequencing file, and a targeted sequencing file sequenced for a specific region, and specifically a whole exome sequencing (WES) file, but is not limited thereto.

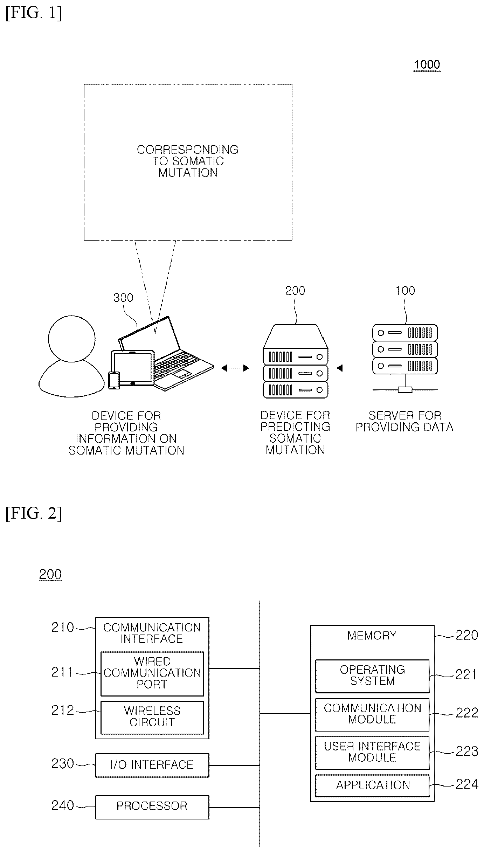

Hereinafter, referring to FIGS. 1 and 2, a system for providing information on the prediction for artificial or somatic mutations based on a device for predicting artificial or somatic mutations according to one embodiment of the present invention and a device for predicting artificial or somatic mutations will be described.

FIG. 1 illustrates a system for providing information on the prediction of artificial or somatic mutations using a device for predicting artificial or somatic mutations according to an embodiment of the present invention.

FIG. 2 illustrates an example of a configuration of a device for predicting artificial or somatic mutations according to an embodiment of the present invention.

First, referring to FIG. 1, a system 1000 for providing information may be a system configured to provide information associated with the prediction of artificial or somatic mutations by applying a deep-learning neural network model based on medical data and/or data obtained from biological samples. At this time, the system 1000 for providing information may consist of a server/device 100 for providing data that provides data obtained from medical data and/or biological samples, a device for predicting artificial or somatic mutations based on the received data, and a device 300 for providing information that visually provides information on the prediction of artificial or somatic mutations in the samples obtained from the individuals based on the received prediction data.

Here, the device 300 for providing information is an electronic device that provides a user interface for displaying information related to the prediction of artificial mutation or somatic mutation, and may include at least one of a smartphone, a tablet personal computer (PC), a laptop, and/or a PC.

The device 300 for providing information on the prediction of somatic mutation may receive information related to the prediction results of artificial or somatic mutations from the device 200 for predicting the somatic mutation and display the received results by a display unit to be described below.

The device 200 for predicting the somatic mutation may include a general-purpose computer, a laptop, and/or a data server that performs various operations to determine information related to the prediction of artificial or somatic mutations based on various data (particularly medical data and/or data on somatic mutation) obtainable from the server/device 100 for providing data. At this time, the server 100 for providing data may be a device for accessing a web server for providing a web page or a mobile web server for providing a mobile web site, but is not limited thereto.

More specifically, the device 200 for predicting the somatic mutation may receive the medical data and/or data on somatic mutation from the server 100 for providing the medical data and predict the artificial or somatic mutations based on the received data, and provide information related thereto.

At this time, the device 200 for predicting the somatic mutation applies a deep-learning neural network model to perform the prediction of artificial or somatic mutations based on the medical data and/or the data on somatic mutation obtained from biological samples, which are received from the server 100 for providing data.

In addition, the device 200 for predicting the somatic mutation may provide prediction results of artificial or somatic mutations to the device 300 for providing information on the prediction of somatic mutation.

As such, the information provided from the device 200 for predicting the somatic mutation may be provided on a web page through a web browser installed in the device 300 for providing information on the prediction of somatic mutation, or provided in the form of an application or program. In various embodiments, such data may be provided in a form included in a platform in a client-server environment.

Hereinafter, the device 200 for predicting the somatic mutation according to an embodiment of the present invention is described as receiving data obtained from the medical data and/or the data obtained from the biological samples from the server 100 for providing data to perform operations, but the device 300 itself for providing information on the prediction of somatic mutation may perform all operations.

Next, components of the device 200 for predicting the somatic mutation of the present invention will be specifically described with reference to FIG. 2.

Referring to FIG. 2, the device 200 for predicting the somatic mutation may include a communication interface 210, a memory 220, an I/O interface 230, and a processor 240, and the respective components may communicate with each other through one or more communication buses or signal lines.

The communication interface 210 may be connected to the device 300 for providing information on the prediction of somatic mutation and the server 100 for providing the medical data via a wired/wireless communication network to exchange data. For example, the communication interface 210 may receive data on somatic mutation and/or individual data from the server 100 for providing the medical data, and may transmit the determined information on the prediction results of artificial or somatic mutations to the device 300 for providing information on the prediction of somatic mutation.

On the other hand, the communication interface 210 to transmit and receive such data includes a communication port 211 and a wireless circuit 212, in which the wired communication port 211 may include one or more wired interfaces, such as Ethernet, a universal serial bus (USB), a firewire, and the like. In addition, the wireless circuit 212 may transmit and receive data with an external device through an RF signal or an optical signal. In addition, wireless communication may use at least one of a plurality of communication standards, protocols and technologies, such as GSM, EDGE, CDMA, TDMA, Bluetooth, Wi-Fi, VoIP, Wi-MAX, or any other suitable communication protocols.

The memory 220 may store various data used in the device 200 for predicting the somatic mutation. For example, the memory 220 may store data on somatic mutation, or store a deep learning model configured to predict artificial or somatic mutations based thereon.

In various embodiments, the memory 220 may include a volatile or nonvolatile recording medium capable of storing various data, instructions, and information. For example, the memory 220 may include at least one type of storage medium of a flash memory type, a hard disk type, a multimedia card micro type, a card type memory (e.g., SD or XD memory, etc.), a RAM, an SRAM, a ROM, an EEPROM, a PROM, a network storage, a cloud, and a blockchain database.

In various embodiments, the memory 220 may store at least one configuration of an operating system 221, a communication module 222, a user interface module 223, and one or more applications 224.

The operating system 221 (e.g., an embedded operating system such as LINUX, UNIX, MAC OS, WINDOWS, VxWorks, etc.) may include various software components and drivers for controlling and managing general system operations (e.g., memory management, storage device control, power management, etc.), and may support communication among various hardware, firmware, and software components.

The communication module 223 may support communication with other devices through the communication interface 210. The communication module 220 may include various software components for processing the data received by the wired communication port 211 or the wireless circuit 212 of the communication interface 210.

The user interface module 223 may receive a request or input of the user from a keyboard, a touch screen, a microphone, etc. through the I/O interface 230, and provide a user interface on a display.

The application 224 may include a program or module configured to be executed by one or more processors 230. Here, the application for providing information related to the prediction of somatic mutation may be implemented on a server farm.

The I/O interface 230 may connect at least one of I/O devices (not illustrated) of the device 200 for predicting the somatic mutation, such as a display, a keyboard, a touch screen and a microphone, with the user interface module 223. The I/O interface 230 may receive a user input (e.g., voice input, keyboard input, touch input, etc.) with the user interface module 223, and process the instructions according to the received input.

The processor 240 is connected to the communication interface 210, the memory 220, and the I/O interface 230 to control the overall operation of the device 200 for predicting the somatic mutation, and may perform various instructions for providing information through an application or program stored in the memory 220.

The processor 240 may correspond to a computing device such as a central processing unit (CPU), a graphic processing unit (GPU) or an application processor (AP). **In** addition, the processor 240 may be implemented in the form of an integrated chip (IC), such as a system on chip (SoC) integrated with various computing devices. Alternatively, the processor 240 may include a module for calculating an artificial neural network model, such as a neural processing unit (NPU).

In various embodiments, the processor 240 may be configured to apply a deep-learning neural network model to generate data necessary for distinguishing between artificial and somatic mutations based on the medical data and/or the data obtained from biological samples and to predict and provide artificial or somatic mutations.

In an embodiment, the device 200 for predicting the somatic mutation may receive medical data from the device 100 for providing the medical data and/or the data obtained from the biological samples derived from the individuals, and apply a deep-learned neural network model using the received data to provide prediction results for the somatic mutation using a model learned to predict artificial or somatic mutations. At this time, the medical data and/or the data obtained from the biological samples derived from the individuals may be data on somatic mutation, and the medical data may be next generation sequencing (NGS) data, but is not limited thereto, and may mean data obtained by observing somatic mutation in biological samples derived from the individuals, but is not limited thereto. Specifically, the NGS data may be at least one of a WGS file, a WES file, an RNA sequencing file, and a target sequencing file, and specifically a WES file, but is not limited thereto.

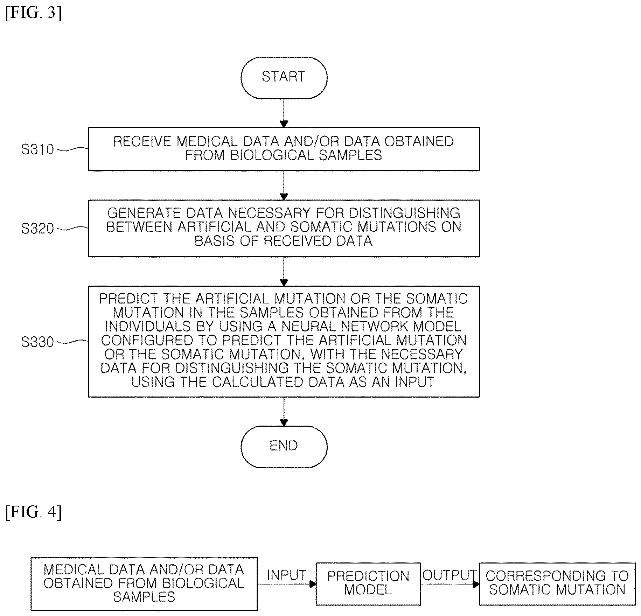

Hereinafter, a method for providing information on the prediction of artificial or somatic mutations according to an embodiment of the present invention will be described in detail with reference to FIGS. 3 and 4.

FIG. 3 is a block diagram of a process of a method for providing information on the prediction of artificial or somatic mutations according to an embodiment of the present invention.

FIG. 4 exemplarily illustrates a process of a method for providing information on artificial or somatic mutations according to various embodiments of the present invention.

First, referring to FIG. 3, a process of the method for providing information on the prediction of artificial or somatic mutations according to an embodiment of the present invention is as follows.

First, medical data and/or data on mutations occurring in biological samples derived from individuals are received (S310).

Next, data necessary for distinguishing between artificial and somatic mutations is generated based on the received data (S320).

The artificial or somatic mutations in the samples obtained from the individuals are predicted by using a neural network model configured to predict artificial or somatic mutations, with the necessary data for distinguishing somatic mutation, using the calculated data as an input(S330).

According to the feature of the present invention, in step (S310) of receiving the medical data and/or the data on mutations occurring in biological samples derived from individuals, a whole exome sequencing (WES) file, an RNA sequencing file, or a targeted sequencing file may be received, and specifically, a WES file may be received, but is not limited thereto.

At this time, the WES files, the RNA sequencing files, and the target sequencing files may have the format of BAM files, but a format of NGS data is not limited thereto.

Next, step (S320) of generating data necessary for distinguishing between artificial and somatic mutations based on the received data may further include vectorizing the received data and calculating a similarity based on the vectorized data.

At this time, referring to Table 1 and FIG. 4 together, in the calculating of the data necessary for distinguishing between artificial and somatic mutations based on the received data, the calculated data may include at least one selected from the group consisting of the number of reads in which a reference sequence is observed/sequence depth at the corresponding position, the number of reads in which an alternate sequence is observed/sequence depth at the corresponding position, a ratio of a difference between the number of sequences in which read1 is mapped to a forward strand among the reads in which the reference sequence is observed and the number of sequences in which read2 is mapped to a reverse strand in the reads in which the reference sequence is observed, a ratio of a difference between the number of sequences in which read1 is mapped to a forward strand among the reads in which the alternate sequence is observed and the number of sequences in which read2 is mapped to a reverse strand in the reads in which the alternate sequence is observed, a value obtained by dividing a median of the inserted length in the read in which the alternate sequence is observed by a median of the inserted length in the read in which the reference sequence is observed, a length of double-sequenced bases by read1 and read2 in a reference fragment, the number of double-sequenced bases by read1 and read2 in the reference fragment, a length of double-sequenced bases by read1 and read2 in an alternate fragment, the number of double-sequenced bases by read1 and read2 in the alternate fragment, a reference sequence (REF_3_BASES) including one base before and after a mutation position, an alternate sequence (ALT_3_BASES) including one base before and after a mutation position, a ratio of a difference between the number of sequences to be mapped to the forward strand among the reads in which the reference sequence is observed and the number of sequences to be mapped to the reverse strand in the reads in which the reference sequence is observed, and a ratio of a difference between the number of sequences to be mapped to the forward strand among the reads in which the alternate sequence is observed and the number of sequences to be mapped to the reverse strand in the reads in which the alternate sequence is observed. In addition, the calculated data may further include a cosine similarity between vectors defined by (the number of sequences in which read1 is mapped to a forward strand among the reads in which the reference sequence is observed and the number of sequences in which read2 is mapped to a reverse strand), or a cosine similarity between a vector defined by (the number of sequences to be mapped to the forward strand among reads in which the reference sequence is observed and the number of sequences to be mapped to the reverse strand) and a vector defined by (the number of sequences to be mapped to the forward strand among reads in which the alternate sequence is observed and the number of sequences to be mapped to the reverse strand), but is not limited thereto.

[Table 1]

| No. | Data associated with distinguishing of somatic mutation |
|---|---|
| 1 | Number of reads in which reference sequence is observed/sequence depth at corresponding position |
| 2 | Number of reads in which alternate sequence is observed/sequence depth at corresponding position |
| 3 | Ratio of difference between fr_ref (number of sequences in which read1 is mapped to forward strand among reads in which reference sequence is observed) and rf_ref (number of sequences in which read2 is mapped to reverse strand) in reads in which reference sequence is observed |
| 4 | Ratio of difference between fr_alt (number of sequences in which read1 is mapped to forward strand among reads in which alternate sequence is observed) and rf_alt (number of sequences in which read2 is mapped to reverse strand) in reads in which alternate sequence is observed |
| 5 | Cosine similarity between vector defined by (fr_ref, rf_ref) and vector defined by (fr_alt, rf_alt) |
| 6 | Value obtained by dividing median of inserted length in read in which alternate sequence is observed by median of inserted length in read in which reference sequence is observed |
| 7 | Length of double-sequenced bases by read1 and read2 in reference fragment |
| 8 | Number of double-sequenced bases by read1 and read2 in reference fragment |
| 9 | Length of double-sequenced bases by read1 and read2 in alternate fragment |

(continued)

| 10 | Number of double-sequenced bases by read1 and read2 in alternate fragment |
|---|---|
| 11 | REF_3_BASES: Reference sequence including 1 base before and after mutation position |
| 12 | ALT_3_BASES: Alternate sequence including 1 base before and after mutation position |
| 13 | Ratio of difference between fs_ref (number of sequences to be mapped to forward strand among reads in which reference sequence is observed) and rs_ref (number of sequences to be mapped to reverse strand) in reads in which reference sequence is observed |
| 14 | Ratio of difference between fs_alt (number of sequences to be mapped to forward strand among reads in which alternate sequence is observed) and rs_alt (number of sequences to be mapped to reverse strand) in reads in which alternate sequence is observed |
| 15 | Cosine similarity between vector defined by (fs_ref, rs_ref) and vector defined by (fs_alt, rs_alt) |

As used herein, the term "read" may mean DNA including an adapter, and the term "fragment" may mean DNA excluding an adapter.

As used herein, the term "read1" means that when Paired End DNA entering NGS equipment is read from both ends, it is read in a 5' to 3' direction, and "read2" means that it is read in a 3' to 5' direction.

As used herein, the term "depth" may be used interchangeably with the term "read-depth" and means the thickness or depth of a read.

As used herein, the term "fr_ref" means the number of sequences in which read1 is mapped to the forward strand among the reads where the reference sequence is observed.

As used herein, the term "rf_ref" means the number of sequences in which read2 is mapped to the reverse strand.

As used herein, the term "fr_alt" means the number of sequences in which read1 is mapped to the forward strand among the reads where the alternate sequence is observed.

As used herein, the term "rf_alt" means the number of sequences in which read2 is mapped to the reverse strand.

As used herein, the term "fs_ref" means the number of sequences to be mapped to the forward strand among the reads where the reference sequence is observed.

As used herein, the term "rs_ref" means the number of sequences to be mapped to the reverse strand.

As used herein, the term "fs_alt" means the number of sequences to be mapped to the forward strand among the reads where the alternate sequence is observed.

As used herein, the term "rs_alt" means the number of sequences to be mapped to the reverse strand.

At this time, a model into which the medical data and/or the data on mutations occurring in the biological samples derived from the individuals may be based on various algorithms such as a Convolution Neural Network (CNN) model, a Deep Neural Network (DNN), a Deep Convolution Neural Network (DCNN), a Recurrent Neural Network (RNN), a Restricted Boltzmann Machine (RBM), a Deep Belief Network (DBN), a Single Shot Detector (SSD), and a Support Vector Machine (SVM), and specifically, may be a CNN model and a DNN model, and more specifically a DNN model, but is not limited thereto.

Next, in step (S330) of predicting the artificial or somatic mutations for the samples obtained from the individuals by using a neural network model configured to predict artificial or somatic mutations, with the necessary data for distinguishing somatic mutation, using the calculated data as an input, the prediction result for the artificial or somatic mutations for the samples obtained from the individuals is determined and provided. For example, the model may be configured to output whether the result value is a true artificial mutation, a false artificial mutation, a true somatic mutation, or a false somatic mutation. For example, the corresponding output may be expressed as a percentage, and a threshold may be set to determine whether a case of having a percentage greater than the threshold is a true artificial mutation or a false artificial mutation, or a true somatic mutation or a false somatic mutation.

At this time, the sample obtained from the individual may be an FFPE sample, and the individual may be an individual developed with any one of liver cancer, colon cancer, breast cancer, lung cancer, and fibrosarcoma, but is not limited thereto.

Meanwhile, the process of providing information on the prediction of artificial or somatic mutations is not limited to those described above.

According to the method for providing information on the prediction of artificial or somatic mutations above, the present invention provides a system for providing information on the prediction of artificial or somatic mutations based on an algorithm, thereby making it easier to set a treatment direction for each individual and contributing to selection of a tailored treatment method.

That is, the present invention can complement the limitations of conventional diagnosis and information provision systems by introducing a system for providing information on the prediction of artificial or somatic mutations based on a neural network model, thereby predicting the artificial or somatic mutations with high accuracy, and more easily setting a treatment direction for each individual.

Example 1: Prediction rate of artificial or somatic mutations in FFPE samples

To generate a trainable dataset, cancer-tissue formalin-fixed paraffin-embedded (FFPE) samples were collected and mutations that also appeared in Frozen Fresh (FF) were predicted from 112,976 mutant colonies.

As a result, it was confirmed that 2,113 mutations were found in both FF and FFPE samples, and 110,863 mutations were found only in the FFPE samples, indicating that only about 1.8% of the mutations were somatic mutation rather than artificial mutation.

Thereafter, features highly correlated with somatic prediction were derived using the obtained samples and a deep-learning neural network model, and then the performance for somatic mutation prediction was evaluated.

Example 2: Evaluation of prediction performance of artificial or somatic mutations in deep-learning neural network model

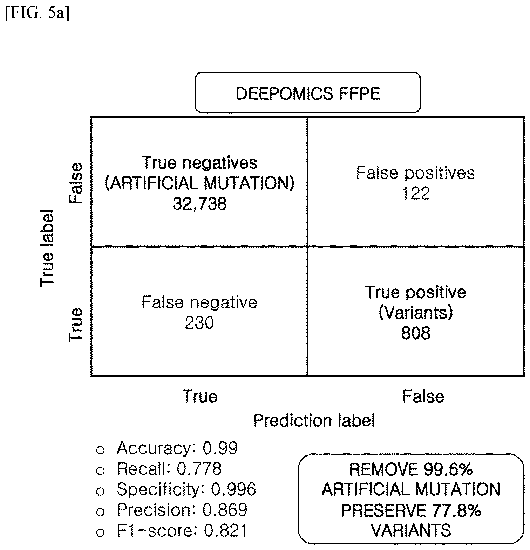

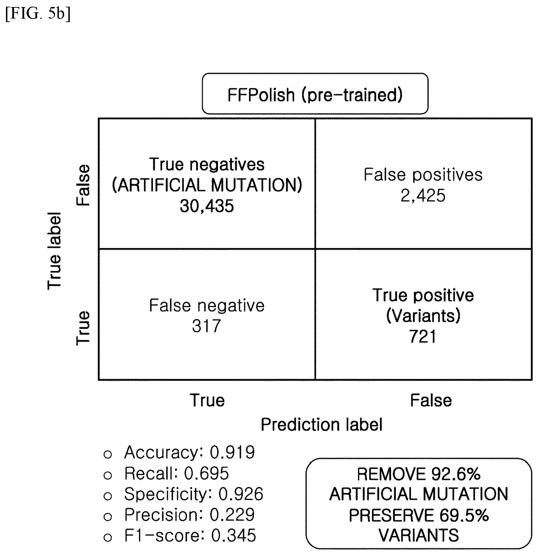

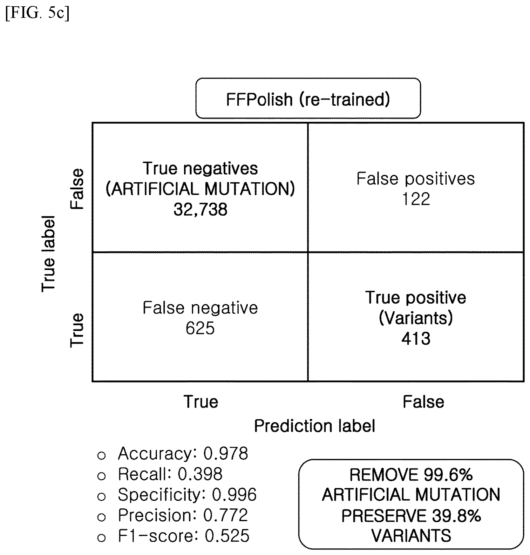

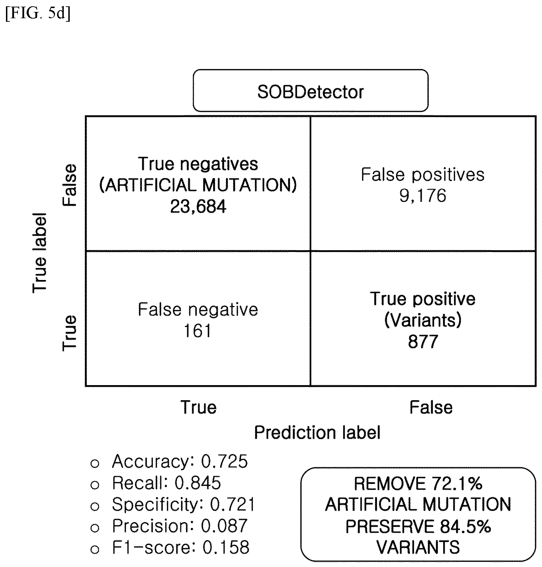

Hereinafter, the performance evaluation results for the prediction of somatic mutation in a deep-learning neural network model according to an embodiment of the present invention will be described with reference to FIG. 5.

FIG. 5a to FIG. 5d are diagrams illustrating results of evaluating the performance of a deep-learning neural network model according to an embodiment of the present invention and Comparative Example.

First, in order to evaluate a deep-learning neural network model according to an embodiment of the present invention, models using various somatic mutation-related data were constructed as Comparative Examples 1 to 3. More specifically, the experiment was conducted by comparing an FFpolish (pre-trained) model as Comparative Example 1, an FFpolish (re-trained) model as Comparative Example 2, and an SOBDetector model as Comparative Example 3 with a neural network model (DEEPOMICS(R) FFPE) of the present invention.

Precision, recall, F1 score, accuracy, and specificity were calculated as follows.

$$precision = \frac{True\,positives}{True\,positives + False\,positives}$$

$$recall = \frac{True\,positives}{True\,positives + False\,negative}$$

$$F1\ score = 2\frac{precision \times recall}{precision + recall}$$

$$Accuracy = \frac{True\,positives + True\,negatives}{True\,positives + True\,negative + False\,positives + False\,negatives}$$

$$Specificity = \frac{True\,negatives}{True\,negatives + False\,positives}$$

As a result, as illustrated in FIG. 5a, it was confirmed that the neural network model (DEEPOMICS® FFPE) according to an embodiment of the present invention removed 99.6% of artificial mutation while preserving 77.8% of actual somatic mutation.

On the other hand, as illustrated in FIG. 5b, in Comparative Example 1 (FFpolish (pre-trained)), it was confirmed to remove 92.6% artificial mutation and preserve 69.5% actual somatic mutation samples, and thus it was confirmed that artificial mutation removal ability and actual somatic mutation sample preservation ability were lower than those of the neural network model according to an embodiment of the present invention.

As illustrated in FIG. 5c, in the case of Comparative Example 2 (FFpolish (re-trained)), the artificial mutation removal rate was 99.6%, showing high removal ability, but it was confirmed that the actual somatic mutation preservation rate was 39.8%. In the case of the model of Comparative Example 2, it was confirmed that when the artificial mutation were removed, samples in which actual somatic mutation occurred were removed together to affect the prediction results.

As illustrated in FIG. 5d, in the case of Comparative Example 3 (SOBDetector), the artificial mutation removal rate was 72.1%, and the actual somatic mutation preservation rate was 84.5%, which was confirmed that the artificial mutation removal rate was significantly low.

When using the neural network model according to an embodiment of the present invention, the F1score was also 0.821, which was confirmed to be superior to other Comparative Examples. As a result as mentioned above, it was confirmed that while the artificial mutation removal ability was excellent, samples in which actual somatic mutation appeared may be not removed but preserved.

Evaluation 2: Measurement of accuracy of prediction of artificial or somatic mutations by cancer type-specific algorithm

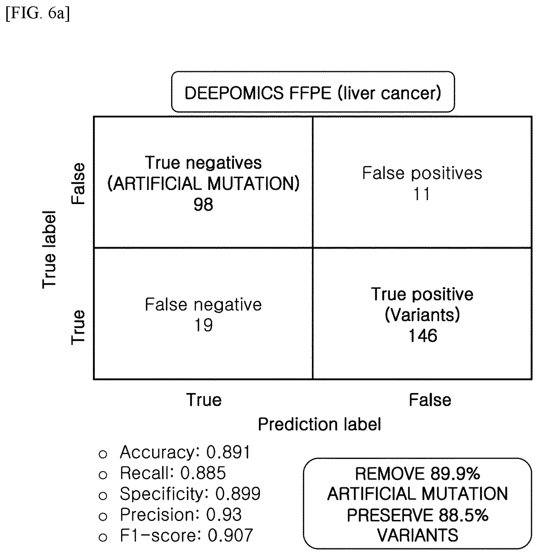

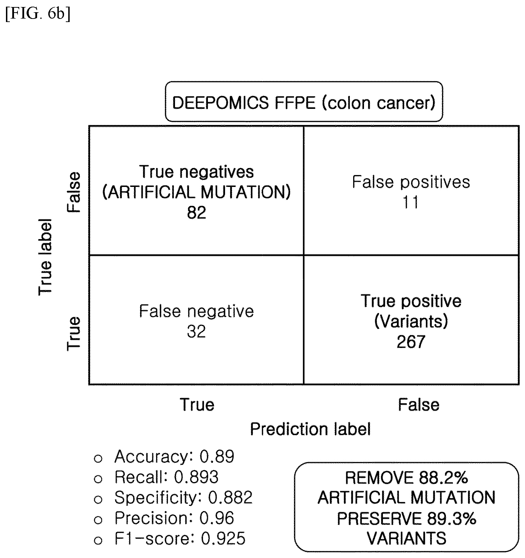

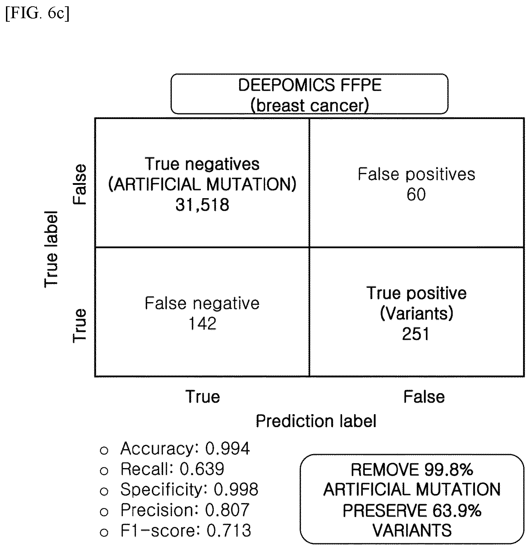

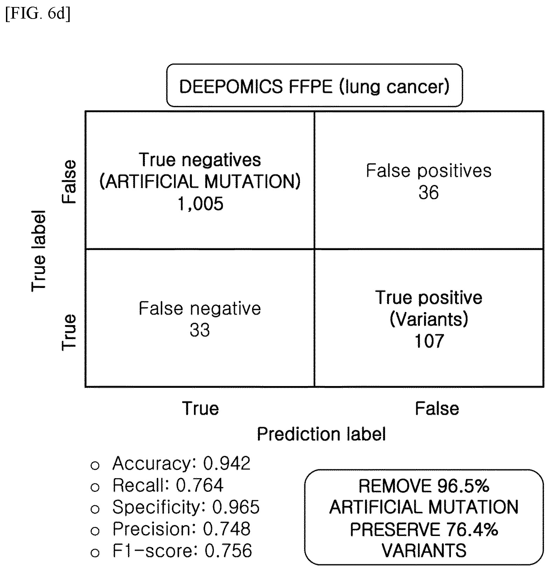

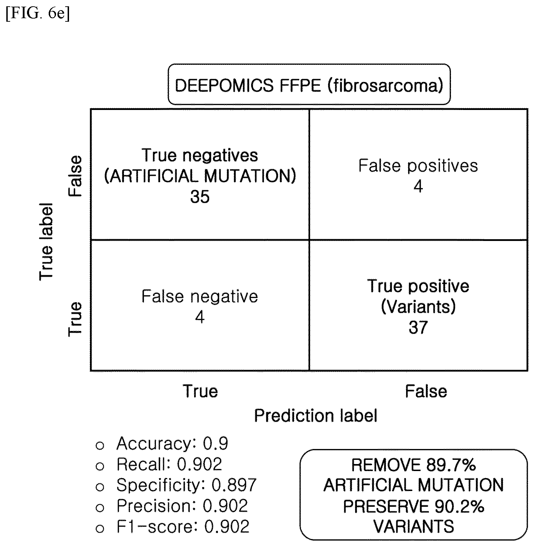

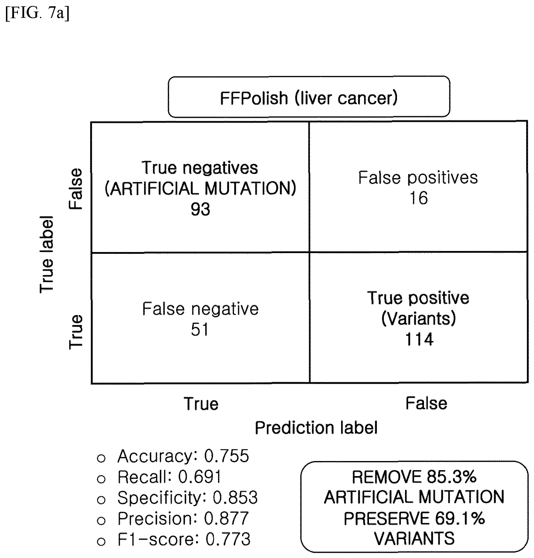

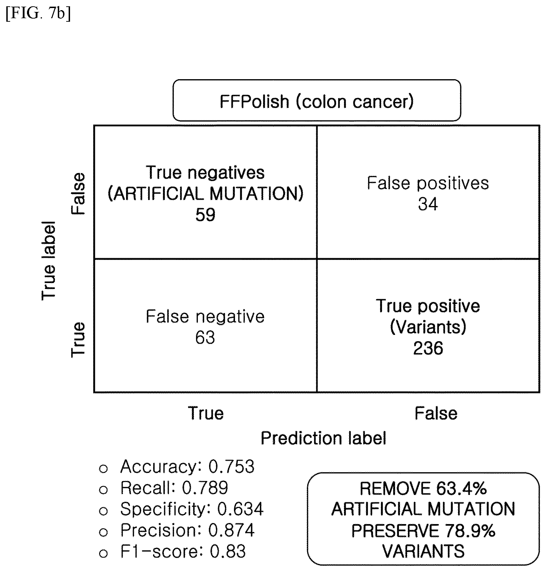

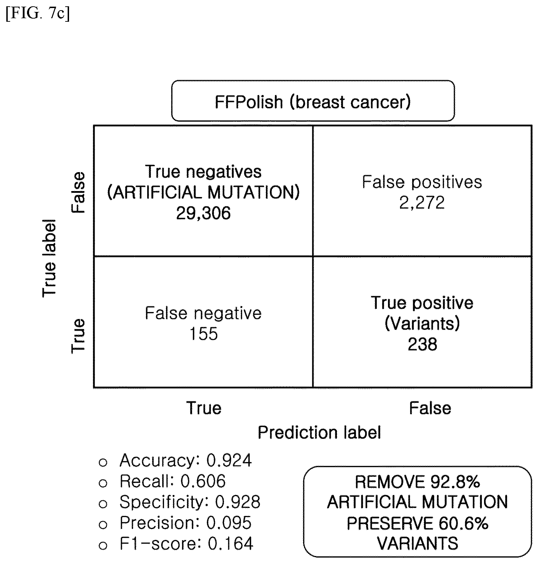

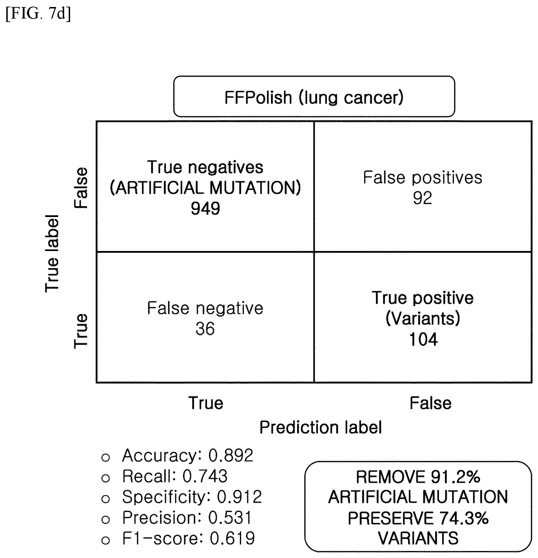

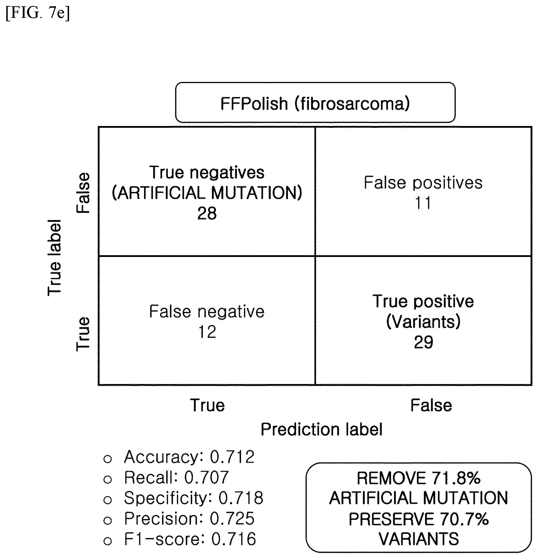

Hereinafter, results of evaluating the performance for the prediction of somatic mutation in various cancer types of a neural network model according to an embodiment of the present invention will be described with reference to FIG. 6a to FIG. 6e and FIG. 7a to FIG. 7e.

FIG. 6a to FIG. 6e illustrate comparisons of the accuracy of the prediction of artificial or somatic mutations for liver cancer, colon cancer, breast cancer, lung cancer, and fibrosarcoma using a neural network model according to an embodiment of the present invention.

FIG. 7a to FIG. 7e illustrate comparisons of the accuracy of the prediction of artificial or somatic mutations for liver cancer, colon cancer, breast cancer, lung cancer, and fibrosarcoma using an FFPolish model of Comparative Example 1 among Comparative Examples of Evaluation 1.

As a result, as illustrated in FIG. 6a to FIG. 6e, when the neural network model according to an embodiment of the present invention was used, in liver cancer, colon cancer, breast cancer, lung cancer, and fibrosarcoma samples, excellent artificial mutation removal ability was shown to be 89.9%, 88.2%, 99.8%, 96.5%, and 89.7%, respectively, and somatic mutation preservation was also shown to be 88.5%, 89.3%, 63.9%, 76.4%, and 90.2%, respectively, and thus it was confirmed that the artificial mutation removal ability was excellent and the preservation ability of actual somatic mutation samples was also excellent.

On the other hand, in the case of the FFpolish model of Comparative Example, as illustrated in FIG. 7a to FIG. 7e, it was confirmed that the artificial mutation removal ability was lower in all samples than that of the model of the present invention, with 85.3% for liver cancer, 63.4% for colon cancer, 92.8% for breast cancer, 91.2% for lung cancer, and 71.8% for fibrosarcoma, respectively, and the somatic mutation sample preservation was 69.1% for liver cancer, 78.9% for colon cancer, 60.6% for breast cancer, 74.3% for lung cancer, and 70.7% for fibrosarcoma, respectively. Thus, it was confirmed that the probability of removing the actual somatic mutation samples while removing the artificial mutation was higher than that of the neural network model according to an embodiment of the present invention.

Although the embodiments of the present invention have been described in detail with reference to the accompanying drawings, the present invention is not limited thereto and may be embodied in many different forms without departing from the technical concept of the present invention. Therefore, the embodiments of the present invention are provided for illustrative purposes only but not intended to limit the technical concept of the present invention. The scope of the technical concept of the present invention is not limited thereto. Therefore, it should be appreciated that the aforementioned embodiments are illustrative in all aspects and are not restricted. The protective scope of the present invention should be construed based on the following claims, and all the technical concepts in the equivalent scope thereof should be construed as falling within the scope of the present invention.

**Claims**

1. A method for providing information on prediction of artificial or somatic mutations, which is implemented by a processor, the method comprising steps of:

   receiving medical data or data obtained from biological samples derived from individuals;
   calculating data necessary for distinguishing between the artificial mutation and the somatic mutation based on the received data; and
   predicting the artificial mutation or the somatic mutation in the samples obtained from the individuals, by using a neural network model configured to predict the artificial mutation or the somatic mutation with the necessary data for distinguishing the somatic mutation, using the calculated data as an input.

2. The method of claim 1, wherein the medical data is next generation sequencing (NGS) data on somatic mutation.

3. The method of claim 2, wherein the NGS data is any one of a whole genome sequencing (WGS) file sequenced for a whole genome, a whole exome sequencing (WES) file, an RNA sequencing file, and a targeted sequencing file sequenced for a specific region.

4. The method of claim 1, wherein in the calculating of the data necessary for distinguishing between the artificial and somatic mutation based on the received data, the calculated data include at least one selected from the group consisting of the number of reads in which a reference sequence is observed/sequence depth at the corresponding position, the number of reads in which an alternate sequence is observed/sequence depth at the corresponding position, a ratio of a difference between the number of sequences in which read1 is mapped to a forward strand among reads in which the reference sequence is observed and the number of sequences in which read2 is mapped to a reverse strand in the reads in which the reference sequence is observed, a ratio of a difference between the number of sequences in which read1 is mapped to a forward strand among reads in which the alternate sequence is observed and the number of sequences in which read2 is mapped to a reverse strand in the reads in which the alternate sequence is observed, a value obtained by dividing a median of an inserted length in the read in which the alternate sequence is observed by a median of an inserted length in the read in which the reference sequence is observed, a length of double-sequenced bases by read1 and read2 in a reference fragment, the number of double-sequenced bases by the read1 and the read2 in the reference fragment, a length of double-sequenced bases by read1 and read2 in an alternate fragment, the number of double-sequenced bases by the read1 and the read2 in the alternate fragment, a reference sequence (REF_3_BASES) including one base before and after a mutation position, an alternate sequence (ALT_3_BASES) including one base before and after the mutation position, a ratio of a difference between the number of sequences to be mapped to the forward strand among the reads in which the reference sequence is observed and the number of sequences to be mapped to the reverse strand in the reads in which the reference sequence is observed, and a ratio of a difference between the number of sequences to be mapped to the forward strand among the reads in which the alternate sequence is observed and the number of sequences to be mapped to the reverse strand in the reads in which the alternate sequence is observed.

5. The method of claim 1, wherein the calculating of the data necessary for distinguishing between the artificial and somatic mutations based on the received data includes vectorizing the received data.

6. The method of claim 5, wherein the calculating of the data necessary for distinguishing between the artificial and somatic mutations based on the received data further includes calculating a similarity based on the vectorized data.

7. The method of claim 4, wherein in the calculating of the data necessary for distinguishing between the artificial and somatic mutations based on the received data, the calculated data further include
a cosine similarity between vectors defined by (the number of sequences in which read1 is mapped to the forward strand among reads in which the reference sequence is observed and the number of sequences in which read2 is mapped to a reverse strand), or a cosine similarity between a vector defined by (the number of sequences to be mapped to the forward strand among the reads in which the reference sequence is observed and the number of sequences to be mapped to the reverse strand) and a vector defined by (the number of sequences to be mapped to the forward strand among reads in which the alternate sequence is observed and the number of sequences to be mapped to the reverse strand).

8. The method of claim 1, wherein in the determining of the prediction for the artificial or somatic mutations in the samples obtained from the individuals based on the calculated data,
the sample obtained from the individual is an FFPE sample.

9. The method of claim 1, wherein in the determining of the prediction for the artificial or somatic mutations in the samples obtained from the individuals based on the calculated data,
the individual is an individual developed with cancer.

10. The method of claim 9, wherein the cancer is any one of liver cancer, colon cancer, breast cancer, lung cancer, and fibrosarcoma.

11. A device for providing information on prediction of artificial or somatic mutations, the device comprising:

a communication unit configured to receive medical data or data on somatic mutation obtained from biological samples from a server for providing data; and
a processor electrically connected with the communication unit,
wherein the processor is configured to
generate data necessary for distinguishing between artificial mutation and somatic mutation based on the received data, and

predict the artificial mutation or somatic mutation in the samples obtained from the individuals by using a neural network model configured to predict the artificial mutation or the somatic mutation, with the necessary data for distinguishing the somatic mutation, using the calculated data as an input.

12. The device of claim 11, wherein the medical data is next generation sequencing (NGS) data on the somatic mutation.

13. The device of claim 12, wherein the NGS data is any one of a whole genome sequencing (WGS) file sequenced for a whole genome, a whole exome sequencing (WES) file, an RNA sequencing file, and a targeted sequencing file sequenced for a specific region.

14. The device of claim 11, wherein the data necessary for distinguishing between the artificial and somatic mutations based on the received data include at least one selected from the group consisting of the number of reads in which a reference sequence is observed/sequence depth at the corresponding position, the number of reads in which an alternate sequence is observed/sequence depth at the corresponding position, a ratio of a difference between the number of sequences in which read1 is mapped to a forward strand among reads in which the reference sequence is observed and the number of sequences in which read2 is mapped to a reverse strand in the reads in which the reference sequence is observed, a ratio of a difference between the number of sequences in which read1 is mapped to a forward strand among reads in which the alternate sequence is observed and the number of sequences in which read2 is mapped to a reverse strand in the reads in which the alternate sequence is observed, a value obtained by dividing a median of an inserted length in the read in which the alternate sequence is observed by a median of an inserted length in the read in which the reference sequence is observed, a length of double-sequenced bases by read1 and read2 in a reference fragment, the number of double-sequenced bases by the read1 and the read2 in the reference fragment, a length of double-sequenced bases by read1 and read2 in an alternate fragment, the number of double-sequenced bases by the read1 and the read2 in the alternate fragment, a reference sequence (REF_3 _BASES) including one base before and after a mutation position, an alternate sequence (ALT_3_BASES) including one base before and after the mutation position, a ratio of a difference between the number of sequences to be mapped to the forward strand among the reads in which the reference sequence is observed and the number of sequences to be mapped to the reverse strand in the reads in which the reference sequence is observed, and a ratio of a difference between the number of sequences to be mapped to the forward strand among the reads in which the alternate sequence is observed and the number of sequences to be mapped to the reverse strand in the reads in which the alternate sequence is observed.

15. The device of claim 11, wherein the processor is further configured to vectorize the received data.

16. The device of claim 15, wherein the processor is further configured to calculate a similarity based on the vectorized data.

17. The device of claim 14, wherein the data necessary for distinguishing between the artificial and somatic mutations based on the received data further include a cosine similarity between vectors defined by (the number of sequences in which read1 is mapped to the forward strand among the reads in which the reference sequence is observed and the number of sequences in which read2 is mapped to the reverse strand), or a cosine similarity between a vector defined by (the number of sequences to be mapped to the forward strand among the reads in which the reference sequence is observed and the number of sequences to be mapped to the reverse strand) and a vector defined by (the number of sequences to be mapped to the forward strand among the reads in which the alternate sequence is observed and the number of sequences to be mapped to the reverse strand).

18. The device of claim 11, wherein the sample obtained from the individual is an FFPE sample.

19. The device of claim 11, wherein the individual is an individual developed with cancer.

20. The device of claim 19, wherein the cancer is any one of liver cancer, colon cancer, breast cancer, lung cancer, and fibrosarcoma.

[FIG. 1]

1000

CORRESPONDING
TO SOMATIC
MUTATION

300

DEVICE FOR
PROVIDING
INFORMATION ON
SOMATIC MUTATION

200

DEVICE FOR
PREDICTING
SOMATIC
MUTATION

100

SERVER FOR
PROVIDING DATA

[FIG. 2]

200

| 210 — COMMUNICATION INTERFACE | MEMORY — 220 |
| 211 — WIRED COMMUNICATION PORT | OPERATING SYSTEM — 221 |
| 212 — WIRELESS CIRCUIT | COMMUNICATION MODULE — 222 |
| 230 — I/O INTERFACE | USER INTERFACE MODULE — 223 |
| 240 — PROCESSOR | APPLICATION — 224 |

[FIG. 3]

```
                    ┌─────────────┐
                    │    START    │
                    └──────┬──────┘
                           │
                           ▼
         ┌────────────────────────────────────────────┐
  S310   │  RECEIVE MEDICAL DATA AND/OR DATA OBTAINED  │
         │         FROM BIOLOGICAL SAMPLES             │
         └───────────────────┬────────────────────────┘
                             │
                             ▼
         ┌────────────────────────────────────────────┐
         │   GENERATE DATA NECESSARY FOR DISTINGUISHING │
  S320   │  BETWEEN ARTIFICIAL AND SOMATIC MUTATIONS ON │
         │            BASIS OF RECEIVED DATA            │
         └───────────────────┬────────────────────────┘
                             │
                             ▼
         ┌────────────────────────────────────────────┐
         │  PREDICT THE ARTIFICIAL MUTATION OR THE SOMATIC │
         │   MUTATION IN THE SAMPLES OBTAINED FROM THE  │
         │  INDIVIDUALS BY USING A NEURAL NETWORK MODEL │
  S330   │  CONFIGURED TO PREDICT THE ARTIFICIAL MUTATION │
         │   OR THE SOMATIC MUTATION, WITH THE NECESSARY │
         │  DATA FOR DISTINGUISHING THE SOMATIC MUTATION, │
         │     USING THE CALCULATED DATA AS AN INPUT    │
         └───────────────────┬────────────────────────┘
                             │
                             ▼
                    ┌─────────────┐
                    │     END     │
                    └─────────────┘
```

[FIG. 4]

```
┌──────────────────────────┐        ┌─────────────┐         ┌──────────────────┐
│ MEDICAL DATA AND/OR DATA │ INPUT  │ PREDICTION  │ OUTPUT  │ CORRESPONDING TO │
│ OBTAINED FROM BIOLOGICAL ├───────▶│   MODEL     ├────────▶│ SOMATIC MUTATION │
│         SAMPLES          │        │             │         │                  │
└──────────────────────────┘        └─────────────┘         └──────────────────┘
```

[FIG. 5a]

| | DEEPOMICS FFPE | |
|---|---|---|
| True negatives (ARTIFICIAL MUTATION) 32,738 | False positives 122 |
| False negative 230 | True positive (Variants) 808 |

True label / False / True (vertical axis)

True — False (Prediction label horizontal axis)

Prediction label

- o Accuracy: 0.99
- o Recall: 0.778
- o Specificity: 0.996
- o Precision: 0.869
- o F1-score: 0.821

REMOVE 99.6% ARTIFICIAL MUTATION PRESERVE 77.8% VARIANTS

[FIG. 5b]

FFPolish (pre-trained)

| | True | False |
|---|---|---|
| **False** (True label) | True negatives (ARTIFICIAL MUTATION) 30,435 | False positives 2,425 |
| **True** (True label) | False negative 317 | True positive (Variants) 721 |

True label

Prediction label: True / False

- o Accuracy: 0.919
- o Recall: 0.695
- o Specificity: 0.926
- o Precision: 0.229
- o F1-score: 0.345

REMOVE 92.6%
ARTIFICIAL MUTATION
PRESERVE 69.5%
VARIANTS

[FIG. 5c]

**FFPolish (re-trained)**

|  | True | False |
|---|---|---|
| **False** | True negatives (ARTIFICIAL MUTATION) 32,738 | False positives 122 |
| **True** | False negative 625 | True positive (Variants) 413 |

True label

True              False

Prediction label

- o Accuracy: 0.978
- o Recall: 0.398
- o Specificity: 0.996
- o Precision: 0.772
- o F1-score: 0.525

REMOVE 99.6%
ARTIFICIAL MUTATION
PRESERVE 39.8%
VARIANTS

[FIG. 5d]

SOBDetector

|  | True | False |
|---|---|---|
| **False** | True negatives (ARTIFICIAL MUTATION) 23,684 | False positives 9,176 |
| **True** | False negative 161 | True positive (Variants) 877 |

True label / Prediction label

- o Accuracy: 0.725
- o Recall: 0.845
- o Specificity: 0.721
- o Precision: 0.087
- o F1-score: 0.158

REMOVE 72.1%
ARTIFICIAL MUTATION
PRESERVE 84.5%
VARIANTS

[FIG. 6a]

DEEPOMICS FFPE (liver cancer)

|  | True negatives (ARTIFICIAL MUTATION) 98 | False positives 11 |
|---|---|---|
| | False negative 19 | True positive (Variants) 146 |

True label — False / True (rows)

Prediction label — True / False (columns)

- o  Accuracy: 0.891
- o  Recall: 0.885
- o  Specificity: 0.899
- o  Precision: 0.93
- o  F1-score: 0.907

REMOVE 89.9%
ARTIFICIAL MUTATION
PRESERVE 88.5%
VARIANTS

[FIG. 6b]

DEEPOMICS FFPE (colon cancer)

|  | True | False |
|---|---|---|
| **False** | True negatives (ARTIFICIAL MUTATION) 82 | False positives 11 |
| **True** | False negative 32 | True positive (Variants) 267 |

True label / Prediction label

o Accuracy: 0.89
o Recall: 0.893
o Specificity: 0.882
o Precision: 0.96
o F1-score: 0.925

REMOVE 88.2%
ARTIFICIAL MUTATION
PRESERVE 89.3%
VARIANTS

[FIG. 6c]

DEEPOMICS FFPE
(breast cancer)

|  | True | False |
|---|---|---|
| **False** | True negatives<br>(ARTIFICIAL MUTATION)<br>31,518 | False positives<br>60 |
| **True** | False negative<br>142 | True positive<br>(Variants)<br>251 |

True label

Prediction label

o Accuracy: 0.994
o Recall: 0.639
o Specificity: 0.998
o Precision: 0.807
o F1-score: 0.713

REMOVE 99.8%
ARTIFICIAL MUTATION
PRESERVE 63.9%
VARIANTS

[FIG. 6d]

DEEPOMICS FFPE (lung cancer)

| | True | False |
|---|---|---|
| **False** | True negatives (ARTIFICIAL MUTATION) 1,005 | False positives 36 |
| **True** | False negative 33 | True positive (Variants) 107 |

True label

True                                    False

Prediction label

o Accuracy: 0.942
o Recall: 0.764
o Specificity: 0.965
o Precision: 0.748
o F1-score: 0.756

REMOVE 96.5%
ARTIFICIAL MUTATION
PRESERVE 76.4%
VARIANTS

[FIG. 6e]

DEEPOMICS FFPE (fibrosarcoma)

|  | True | False |
|---|---|---|
| **False** | True negatives (ARTIFICIAL MUTATION) 35 | False positives 4 |
| **True** | False negative 4 | True positive (Variants) 37 |

True label / Prediction label

o Accuracy: 0.9
o Recall: 0.902
o Specificity: 0.897
o Precision: 0.902
o F1-score: 0.902

REMOVE 89.7%
ARTIFICIAL MUTATION
PRESERVE 90.2%
VARIANTS

[FIG. 7a]

FFPolish (liver cancer)

|  | True negatives (ARTIFICIAL MUTATION) 93 | False positives 16 |
|  | False negative 51 | True positive (Variants) 114 |

True label — False / True

Prediction label — True / False

o Accuracy: 0.755
o Recall: 0.691
o Specificity: 0.853
o Precision: 0.877
o F1-score: 0.773

REMOVE 85.3%
ARTIFICIAL MUTATION
PRESERVE 69.1%
VARIANTS

[FIG. 7b]

FFPolish (colon cancer)

|  | True | False |
|---|---|---|
| **False** | True negatives (ARTIFICIAL MUTATION) 59 | False positives 34 |
| **True** | False negative 63 | True positive (Variants) 236 |

True label / Prediction label

o Accuracy: 0.753
o Recall: 0.789
o Specificity: 0.634
o Precision: 0.874
o F1-score: 0.83

REMOVE 63.4%
ARTIFICIAL MUTATION
PRESERVE 78.9%
VARIANTS

[FIG. 7c]

| | FFPolish (breast cancer) | |
|---|---|---|
| **False** | True negatives (ARTIFICIAL MUTATION) 29,306 | False positives 2,272 |
| **True** | False negative 155 | True positive (Variants) 238 |
| | True | False |

True label

Prediction label

- o Accuracy: 0.924
- o Recall: 0.606
- o Specificity: 0.928
- o Precision: 0.095
- o F1-score: 0.164

REMOVE 92.8%
ARTIFICIAL MUTATION
PRESERVE 60.6%
VARIANTS

[FIG. 7d]

FFPolish (lung cancer)

| | True label | True negatives (ARTIFICIAL MUTATION) 949 | False positives 92 |
|---|---|---|---|
| | False | | |
| | True | False negative 36 | True positive (Variants) 104 |

True label

Prediction label: True — False

- o Accuracy: 0.892
- o Recall: 0.743
- o Specificity: 0.912
- o Precision: 0.531
- o F1-score: 0.619

REMOVE 91.2%
ARTIFICIAL MUTATION
PRESERVE 74.3%
VARIANTS

[FIG. 7e]

FFPolish (fibrosarcoma)

|  | True negatives (ARTIFICIAL MUTATION) 28 | False positives 11 |
|---|---|---|
|  | False negative 12 | True positive (Variants) 29 |

True label (False / True)

True          False
Prediction label

o Accuracy: 0.712
o Recall: 0.707
o Specificity: 0.718
o Precision: 0.725
o F1-score: 0.716

REMOVE 71.8%
ARTIFICIAL MUTATION
PRESERVE 70.7%
VARIANTS

## INTERNATIONAL SEARCH REPORT

| | International application No. |
|---|---|
| | **PCT/KR2023/012761** |

**A. CLASSIFICATION OF SUBJECT MATTER**

**G16B 40/20**(2019.01)i; **G16B 20/20**(2019.01)i; **G16B 30/10**(2019.01)i; **G16B 45/00**(2019.01)i; **C12Q 1/6886**(2018.01)i; **G16B 40/00**(2019.01)i

According to International Patent Classification (IPC) or to both national classification and IPC

**B. FIELDS SEARCHED**

Minimum documentation searched (classification system followed by classification symbols)

G16B 40/20(2019.01); C12Q 1/6869(2018.01); C12Q 1/6886(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 체세포 변이(somatic mutation), 인공 변이(artifactual mutation), 딥 러닝(deep learning), FFPE 샘플(FFPE sample), NGS 데이터(NGS data)

**C. DOCUMENTS CONSIDERED TO BE RELEVANT**

| Category* | Citation of document, with indication, where appropriate, of the relevant passages | Relevant to claim No. |
|---|---|---|
| X | KIM, Hyunbin et al. FIREVAT: finding reliable variants without artifacts in human cancer samples using etiologically relevant mutational signatures. Genome Medicine. 2019, vol. 11, article no. 81, pp. 1-17. See abstract; pages 2-3, 11, 13 and 15; and figures 1 and 4-5. | 1-20 |
| X | DODANI, Dollina D. et al. Combinatorial and machine learning approaches for improved somatic variant calling from formalin-fixed paraffin-embedded genome sequence data. Frontiers in Genetics. 27 April 2022, vol. 13, article no. 834764, pp. 1-9. See abstract; pages 2 and 4-7; and figure 2. | 1-4,8-14,18-20 |
| X | WOOD, Derrick E. et al. A machine learning approach for somatic mutation discovery. Science Translational Medicine. 2018, vol. 10, eaar7939, pp. 1-16. See abstract; pages 2-3 and 7-12; and figure 1. | 1-3,8-13,18-20 |
| A | WU, Chao et al. Using machine learning to identify true somatic variants from next-generation sequencing. Clinical Chemistry. 2020, vol. 66, no. 1, pp. 239-246. See entire document. | 1-20 |

✓ Further documents are listed in the continuation of Box C.    ✓ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 December 2023** | **18 December 2023** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 632 747 A1**

## INTERNATIONAL SEARCH REPORT

| | International application No. |
|---|---|
| | **PCT/KR2023/012761** |

### C. DOCUMENTS CONSIDERED TO BE RELEVANT

| Category* | Citation of document, with indication, where appropriate, of the relevant passages | Relevant to claim No. |
|---|---|---|
| A | DE SCHAETZEN VAN BRIENEN, Louise et al. Comparative analysis of somatic variant calling on matched FF and FFPE WGS samples. BMC Medical Genomics. 2020, vol. 13, article no. 94, pp. 1-15.<br>See entire document. | 1-20 |
| A | CN 112029861 A (ZHENYUE BIOTECHNOLOGY JIANGSU CO., LTD. et al.) 04 December 2020 (2020-12-04)<br>See entire document. | 1-20 |
| PX | HEO, Dong-Hyuk et al. Reducing artifactual somatic variant calls from formalin-fixed paraffin-embedded specimens by using DEEPOMICS FFPE, a bioinformatic approach based on deep neural networks. Research Square. 22 June 2023, pp. 1-20. Retrieved from <URL: https://doi.org/10.21203/rs.3.rs-2922419/v2>.<br>See abstract; pages 5-13; and figure 1. | 1-3,8-13,18-20 |

Form PCT/ISA/210 (second sheet) (July 2022)

33

## INTERNATIONAL SEARCH REPORT
### Information on patent family members

International application No.

**PCT/KR2023/012761**

| Patent document cited in search report | | | Publication date (day/month/year) | Patent family member(s) | | | Publication date (day/month/year) |
|---|---|---|---|---|---|---|---|
| CN | 112029861 | A | 04 December 2020 | CN | 112038378 | A | 04 December 2020 |
| | | | | US | 2022-0072553 | A1 | 10 March 2022 |
| | | | | US | 2022-0320181 | A1 | 06 October 2022 |
| | | | | WO | 2022-047911 | A1 | 10 March 2022 |
| | | | | WO | 2022-047929 | A1 | 10 March 2022 |
| | | | | WO | 2022-048106 | A1 | 10 March 2022 |

Form PCT/ISA/210 (patent family annex) (July 2022)